# EUROPEAN PATENT SPECIFICATION

(11) **EP 2 506 824 B1**
(45) Date of publication and mention of the grant of the patent: **31.05.2017**
(21) Application number: 09768291.8
(22) Date of filing: 04.12.2009
(51) Int. Cl.: A61K 8/60, A61K 8/66, A61Q 11/00

(54) **NON-AQUEOUS, SINGLE TUBE DENTRIFICE WHITENING COMPOSITIONS, METHODS OF USE AND MANUFACTURE THEREOF**
WASSERFREIE AUFHELLENDE ZAHNPASTAZUSAMMENSETZUNGEN IN EINER TUBE, ANWENDUNGSVERFAHREN UND HERSTELLUNGSVERFAHREN DAFÜR
COMPOSITIONS BLANCHISSANTES NON AQUEUSES DE DENTIFRICE, EN TUBE UNIQUE, PROCÉDÉS D'UTILISATION ET DE FABRICATION DE CELLES-CI

(43) Date of publication of application: 10.10.2012
(73) Proprietor: Colgate-Palmolive Company, New York, NY 10022 (US)
(72) Inventor: CHOPRA, Suman, K., Monroe, NJ 08831 (US); ZAIDEL, Lynette, Cranford, NJ 07016 (US); IBRAHIM, Sayed, Somerset, NJ 08873 (US); LEIGH, Leonora, Piscataway, NJ 08854 (US); PRENCIPE, Michael, West Windsor, NJ 08550 (US)
(74) Representative: Wibbelmann, Jobst
(86) International application number: PCT/US2009/066737
(87) International publication number: WO 2011/068514

(56) References cited:
- EP-A2- 0 133 736
- WO-A1-91/11105
- WO-A1-97/41215
- US-A- 4 178 362
- US-A- 4 590 065

## Description

### BACKGROUND OF THE INVENTION

Stain can be removed from tooth surfaces by the use of dentifrices, especially toothpaste, gels and powders containing active oxygen or hydrogen peroxide-liberating ingredients such as peroxides, percarbonates and perborates. However, these methods do not fulfill the needs of individuals who desire rapid whitening of teeth.

Some conventional at-home whitening methods utilize a tray containing a bleaching agent placed upon the teeth of the patient and bleaching is allowed to take place. This method of treatment has drawbacks including tooth sensitivity, possibly due to demineralization and irritation of oral tissues.

One of the drawbacks to home use bleaching products containing oxygen liberating-bleaching compounds is the tendency of these products to decompose within a relatively short period of time following manufacture with concomitant loss of all or a substantial amount of the available oxygen, thereby limiting the efficacy of these products as teeth whitening compositions. Peroxy compounds such as hydrogen peroxide are unstable with respect to maintenance of peroxide level and have been found to be difficult to formulate into aqueous gels or pastes, which will have an adequate shelf-life and yet will readily liberate oxygen when applied to the oral cavity. Certain formulations of oxygen liberating-compositions for the whitening of teeth utilize anhydrous powders or water-free pastes or gels, which must be protected against contamination and chemical interaction. A drawback to the use of such anhydrous products is that, due to the absence of water, application of the oral composition tends to desiccate oral tissues, which may lead to irritation and tissue damage.

EP-A-0133736 discloses a di-enzymatic dentifrice. WO-A-91/11105 discloses anti-microbial compositions. US 4,178,362 discloses enzymatic dentifrices. WO-A-97/41215 discloses non-aqueous, liquid, enzyme-containing compositions. US 4,590,065 discloses a stable flavor-containing dentifrice.

### BRIEF SUMMARY OF THE INVENTION

The present invention relates to oral care products and methods of using the same that are effective in whitening a tooth surface and removing or reducing the accumulation of calculus and preventing gingivitis.

The present invention provides an oral care product in the form of a stable, non-aqueous dentifrice composition according to claim 1.

Preferred features are defined in the dependent claims.

The present invention also provides a stable, non-aqueous dentifrice composition according to the present invention for use in whitening of a tooth surface, or for use in the treatment of oral calculus accumulation.

### DETAILED DESCRIPTION OF THE INVENTION

As used throughout, ranges are used as a shorthand for describing each and every value that is within the range. Any value within the range can be selected as the terminus of the range. In the event of a conflict in a definition in the present disclosure and that of a cited reference, the present disclosure controls.

In one embodiment the invention provides a stable, non-aqueous dentifrice composition comprising at least one enzyme, at least one substrate, and a suitable carrier. As used herein and unless otherwise indicated, the term "suitable carrier" refers to any agent or combination of agents including, but not limited to, surfactants, polymers, and copolymers that form a non-aqueous system, which allows the enzyme and substrate to maintain in proximity in a stable manner. As used herein and unless otherwise indicated, the term "substrate" refers to any agent that when in contact with an enzyme generates hydrogen peroxide. An example of an enzyme and substrate that generates hydrogen peroxide is glucose oxidase and glucose.

In certain embodiments of the invention the compositions may also include one or more additional agents. In other embodiments, the compositions further include at least one abrasive. In another embodiment, the compositions further include at least one fluoride source. In another embodiment, the compositions further include at least one polymer or copolymer. In another embodiment, the compositions further include at least one surfactant. In another embodiment, the compositions further include at least one vitamin. In another embodiment, the compositions further include at least one flavoring agent. In another embodiment, the compositions further include at least one enzyme. In another embodiment, the compositions further include at least one humectant. In another embodiment, the compositions further include at least one preservative.

In certain embodiments of the invention, the non-aqueous dentifrice composition is contained in a single container such as a single tube.

In certain embodiments of the invention, the abrasive includes, but is not limited to, abrasive silica, sodium metaphosphate, potassium metaphosphate, tricalcium phosphate, dihydrated dicalcium phosphate, aluminum silicate, calcined alumina, bentonite or other siliceous materials, or combinations thereof.

In another embodiment of the invention, the fluoride source includes, but is not limited to, sodium fluoride, potassium fluoride, sodium fluorosilicate, sodium monfluorophosphate (MFP), ammonium fluorosilicate, stannous fluoride and stannous chloride.

In another embodiment of the invention, the polymer or copolymer includes, but is not limited to, polyethylene glycol, a PEG/PPG copolymer, PVP, and/or polyethylene.

In another embodiment of the invention, the flavoring agent includes, but is not limited to, oil of wintergreen, oil of peppermint, oil of spearmint, oil of sassafras, oil of clove, aspartame, acesulfame, saccharin, dextrose, levulose and sodium cyclamate and combinations thereof.

The invention also encompasses methods of whitening a tooth surface and/or reducing or removing calculus from the oral cavity including contacting the tooth surface or oral cavity with a stable, non-aqueous dentifrice composition of the invention, which comprises at least one enzyme, at least one substrate, and a suitable carrier.

The compositions of the invention include one or more enzymes. The enzymes of the invention serve to interact with a substrate to generate hydrogen peroxide. Useful enzymes include any of the available oxidases, which when contacted with a substrate interact to generate hydrogen peroxide.

In certain embodiments, the enzyme is a glucose oxidase, monoamine oxidase, xanthine oxidase, gluconolactone oxidase, laccase, lysyl oxidase, or combinations thereof.

An enzyme or a mixture of several compatible enzymes in the current invention is present in the invention in an amount of 0.002% to 2% by weight, of 0.05% to 1.5% weight, or of 0.1% to 0.5% by weight, these amounts denoting % by weight of the composition.

The substrates of the invention include any substance that when contacted with an appropriate enzyme releases hydrogen peroxide. An example of a substrate includes glucose, which when contacted with glucose oxidase generates hydrogen peroxide.

The compositions may optionally include one or more abrasives including, but are not limited to, silica abrasives such as precipitated silicas having a mean particle size of up to 20 microns, such as Zeodent 115^{®}, marketed by J. M. Huber. Useful abrasives also include sodium metaphosphate, potassium metaphosphate, tricalcium phosphate, dihydrated dicalcium phosphate, aluminum silicate, calcined alumina, bentonite or other siliceous materials, or combinations thereof. The silica abrasive polishing materials useful herein, as well as the other abrasives, generally have an average particle size of 0.1 to 30 microns, or 5 to 15 microns. The silica abrasives can be from precipitated silica or silica gels, such as the silica xerogels described in U.S. Pat. No. 3,538,230, to Pader et al. and U.S. Pat. No. 3,862,307, to Digiulio. Particular silica xerogels are marketed under the trade name Syloid^{®} by the W. R. Grace & Co., Davison Chemical Division. The precipitated silica materials include those marketed by the J. M. Huber Corp. under the trade name Zeodent^{®}, including the silica carrying the designation Zeodent^{®} 115 and 119. These silica abrasives are described in U.S. Pat. No. 4,340,583, to Wason.

In certain embodiments, abrasive materials include silica gels and precipitated amorphous silica having an oil absorption value of less than 100 cc/100 g silica and in the range of 45 cc/100 g to 70 cc/100 g silica, 45 cc/100 g to 65 cc/100 g silica, or 45 cc/100 g to 60 cc/100 g silica. Oil absorption values are measured using the ASTA Rub-Out Method D281. In other embodiments, the silicas are colloidal particles having an average particle size of 3 microns to 12 microns, and 5 to 10 microns.

Low oil absorption silica abrasives particularly useful in the practice of the invention are marketed under the trade designation Sylodent XWA^{®} by Davison Chemical Division of W.R. Grace & Co., Baltimore, Md. 21203. Sylodent 650 XWA^{®}, a silica hydrogel composed of particles of colloidal silica having a water content of 29% by weight averaging 7 to 10 microns in diameter, and an oil absorption of less than 70 cc/100 g of silica is an example of a low oil absorption silica abrasive useful in the practice of the present invention. The abrasive is present in the oral care strip or tape composition of the present invention at a concentration of 1 to 40% by weight of the composition, in other embodiment 5 to 30% by weight, and in another embodiment 10 to 20% by weight. The dosage of abrasive in the individual strip or tape (i.e., a single dose) is 0.01 to 0.4% by weight of the composition, 0.05 to 0.3% by weight, and in another embodiment 0.1 to 0.2 % by weight.

The compositions may also include one or more fluoride ion sources. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al.

Representative fluoride ion sources include, but are not limited to, stannous fluoride, sodium fluoride, potassium fluoride, sodium monofluorophosphate, sodium fluorosilicate, sodium monfluorophosphate (MFP), ammonium fluorosilicate, as well as tin fluorides, such as stannous fluoride and stannous chloride, and combinations thereof. Certain particular embodiments include stannous fluoride or sodium fluoride as well as mixtures thereof.

In certain embodiments, the compositions of the invention may also contain a source of fluoride ions or fluorine-providing ingredient in amounts sufficient to supply 25 ppm to 5,000 ppm of fluoride ions.

Fluoride ion sources may be added to the compositions of the invention at a level of 0.01% to 3% in one embodiment or 0.03% to 1%, by weight of the composition, in another embodiment. The dosage of the individual strip or tape (i.e., a single dose) may be 0.0001 to 0.003% by weight of the composition, 0.0005 to 0.003% by weight, and in another embodiment 0.001 to 0.02% by weight.

Another agent included in the compositions of the invention is a surfactant or a mixture of compatible surfactants. Suitable surfactants are those which are reasonably stable throughout a wide pH range, for example, anionic, cationic, nonionic or zwitterionic surfactants.

Suitable surfactants are described more fully, for example, in U.S. Pat. No. 3,959,458, to Agricola et al.; U.S. Pat. No. 3,937,807, to Haefele; and U.S. Pat. No. 4,051,234, to Gieske et al.

In certain embodiments, the anionic surfactants useful herein include the water-soluble salts of alkyl sulfates having from 10 to 18 carbon atoms in the alkyl radical and the water-soluble salts of sulfonated monoglycerides of fatty acids having from 10 to 18 carbon atoms. Sodium lauryl sulfate, sodium lauroyl sarcosinate and sodium coconut monoglyceride sulfonates are examples of anionic surfactants of this type. Mixtures of anionic surfactants may also be utilized.

In another embodiment, cationic surfactants useful in the present invention can be broadly defined as derivatives of aliphatic quaternary ammonium compounds having one long alkyl chain containing 8 to 18 carbon atoms such as lauryl trimethylammonium chloride, cetyl pyridinium chloride, cetyl trimethylammonium bromide, di-isobutylphenoxyethyldimethylbenzylammonium chloride, coconut alkyltrimethylammonium nitrite, cetyl pyridinium fluoride, and mixtures thereof.

Illustrative cationic surfactants are the quaternary ammonium fluorides described in U.S. Pat. No. 3,535,421, to Briner et al. Certain cationic surfactants can also act as germicides in the compositions.

Illustrative nonionic surfactants that can be used in the compositions of the invention can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

In certain embodiments, zwitterionic synthetic surfactants useful in the present invention can be broadly described as derivatives of aliphatic quaternary ammonium, phosphonium, and sulfonium compounds, in which the aliphatic radicals can be straight chain or branched, and wherein one of the aliphatic substituents contains 8 to 18 carbon atoms and one contains an anionic water-solubilizing group, e.g., carboxy, sulfonate, sulfate, phosphate or phosphonate. Illustrative examples of the surfactants suited for inclusion into the composition include, but are not limited to, sodium alkyl sulfate, sodium lauroyl sarcosinate, cocoamidopropyl betaine and polysorbate 20, and combinations thereof.

The surfactant or mixtures of compatible surfactants can be present in the compositions of the present invention 0.1 % to 5%, in another embodiment 0.3% to 3% and in another embodiment 0.5% to 2% by weight of the total composition. The dosage of surfactant in the individual strip or tape (i.e., a single dose) is 0.001 to 0.05% by weight of the composition, 0.003 to 0.03% by weight, and in another embodiment 0.005 to 0.02 % by weight.

The compositions of the invention may also include a flavoring agent. Flavoring agents which are used in the practice of the present invention include, but are not limited to, essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

The flavoring agent is incorporated in the oral composition at a concentration of 0.1 to 5% by weight of the composition, or 0.5 to 1.5% by weight. The dosage of flavoring agent in the individual strip or tape (i.e., a single dose) is 0.001 to 0.05% by weight of the composition and in another embodiment 0.005 to 0.015 % by weight.

The compositions of the invention also may optionally include one or more chelating agents able to complex calcium found in the cell walls of bacteria. Binding of this calcium weakens the bacterial cell wall and augments bacterial lysis.

Another group of agents suitable for use as chelating agents in the present invention are the soluble pyrophosphates. The pyrophosphate salts used in the present compositions can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The salts are useful in both their hydrated and unhydrated forms. An effective amount of pyrophosphate salt useful in the present composition is generally enough to provide at least 1.0% pyrophosphate ions by weight of the composition, 1.5% to 6%, 3.5% to 6% of such ions. The dosage chelating agent in the individual strip or tape (i.e., a single dose) is 0.01 to 0.6% by weight of the composition and in another embodiment 0.035 to 0.06 % by weight.

The compositions of the invention also optionally include one or more polymers. Such materials are well known in the art, being employed in the form of their free acids or partially or fully neutralized water soluble alkali metal (e.g., potassium and sodium) or ammonium salts. Certain embodiments include 1:4 to 4:1 copolymers of maleic anhydride or acid with another polymerizable ethylenically unsaturated monomer, for example, methyl vinyl ether (methoxyethylene) having a molecular weight (M.W.) of 30,000 to 1,000,000. These copolymers are available for example as Gantrez AN 139(M.W. 500,000), AN 119 (M.W. 250,000) and S-97 Pharmaceutical Grade (M.W. 70,000), of GAF Chemicals Corporation.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone.

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight from 1,000-2,000,000, described in U.S. Pat. No. 4,842,847, Jun. 27,1989 to Zahid.

Another useful class of polymeric agents includes polyamino acids, particularly those containing proportions of anionic surface-active amino acids such as aspattic acid, glutamic acid and phosphoserine, as disclosed in U.S. Pat. No. 4,866,161 Sikes et al.

In preparing compositions, it is sometimes necessary to add some thickening material to provide a desirable consistency. In certain embodiments, the thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate or finely divided silica can be used as component of the thickening composition to further improve the composition's texture. Thickening agents in an amount from 0.5% to 5.0% by weight of the total composition can be used.

Within oral compositions, it is also desirable to incorporate a humectant to prevent the composition from hardening upon exposure to air. Certain humectants can also impart desirable sweetness or flavor to dentifrice compositions. The humectant, on a pure humectant basis, generally includes 15% to 70% in one embodiment or 30% to 65% in another embodiment by weight of the dentifrice composition.

Suitable humectants include edible polyhydric alcohols such as glycerine, sorbitol, xylitol, propylene glycol as well as other polyols and mixtures of these humectants. Mixtures of glycerine and sorbitol may be used in certain embodiments as the humectant component of the toothpaste compositions herein.

In addition to the above described components, the embodiments of this invention can contain a variety of optional dentifrice ingredients some of which are described below. Optional ingredients include, for example, but are not limited to, adhesives, sudsing agents, flavoring agents, sweetening agents, additional antiplaque agents, abrasives, and coloring agents. These and other optional components are further described in U.S. Pat. No. 5,004,597, to Majeti; U.S. Pat. No. 3,959,458 to Agricola et al. and U.S. Pat. No. 3,937,807, to Haefele.

The compositions of the present invention can be made using methods which are common in the oral product area.

In one illustrative embodiment, the non-aqueous oral care composition is made by combining Pluracare L-1220, L-4370, and PEG 600, which were mixed in Mini Ross pot for 15 minutes under full vacuum. Saccharin and sodium fluoride were added and the mixture was mixed for 5 minutes under full vacuum. Then high cleaning silica and fumed silica were added and the mixture was mixed for another 15 minutes. Flavor, glucose oxidase, and glucose were then added and the mixture was mixed for 15 minutes.

The following examples further describe and demonstrate illustrative embodiments within the scope of the present invention. The examples are given solely for illustration and are not to be construed as limitations of this invention as many variations are possible without departing from the scope thereof.

### EXPERIMENTAL EXAMPLES

### Example 1

Example 1 illustrates illustrative embodiments of the compositions of the single tube, non-aqueous dentifrice composition various ranges in which each ingredient may be contained in an illustrative embodiment.

| **Ingredient** | **% in dentifrice** |
|---|---|
| **Pluracare L-1220** | 0.1-99 |
| **Enzyme** | 0.01-30 |
| **Substrate, Glucose** | 0.01-20 |
| **Abrasive** | 0-40 |
| **Pluracare L-4370** | 0-80 |
| **Phosphate** | 0-20 |
| **Fluoride** | 0-2 |
| **DC Silicone Fluid - 350CST** | 0-50 |
| **Plastigel 5** | 0-50 |
| **Thickener (e.g., xPVP powder)** | 0.01-50 |
| **Fumed Silica** | 0.1-10 |
| **Viscosity** | **10,000-700,000 cps** |

### Example 2

Example 2 illustrates a single tube prototype and compares this with a conventional dual tube arrangement and measures the amount of peroxide released.

| **Prototype** | **Amount of Enzyme** | **Amount of Substrate** | **Micromoles of Peroxide** |
|---|---|---|---|
| Single Tube (nonaqueous) | 0.25% GOX | 1.5 % Glucose | 46 |
| Double Tube (aqueous) | 0.32 % GOX | 1.5 % Glucose | 27 |

The single tube product (formula 1) containing GOX/Glucose was analyzed for peroxide availability by measuring the amount of peroxide generated by the sample. The product was compared to an aqueous dual tube product in which GOX and glucose were separated from each other. The results are shown below. Surprisingly, the data shows that the single tube product generates more peroxide than the conventional dual tube arrangement.

### Example 3

Example 3 illustrates various illustrative embodiments of the stable, non-aqueous, single tube compositions of the invention. It was discovered that the use of non-aqueous dentifrice compositions prevent interaction between incompatible components GOX and glucose in the product. In certain embodiments, a solid or non-aqueous composition of GOX was used in PEG, glycerin, and PG. Also different molecular weights of pluronics with ethylene and propylene groups. In addition, non-bitter liquid copolymers of ethylene oxide and propylene oxides, such as BASF Pluracare L4370 and L1220 can be used.

| **Ingredient** | **Description** | **Formula 1** | **Formula 2** | **Formula 3** | **Formula 4** | **Formula 5** |
|---|---|---|---|---|---|---|
| **Pluracare L4370** | PEG/PPG 38/8 copolymer | 10 | 57.96 | 50.31 | 44.81 | 44.81 |
| **Pluracare L1220** | PEG/PPG 116/66 copolymer | 57.76 | 10 | 10 | 10 | 10 |
| **PEG 600** | Polyethylene glycol | 10 | 10 | 5 | 5 | 5 |
| **Saccharin** | Sodium saccharin | 0.3 | 0.3 | 0.3 | 0.3 | 0.3 |
| **NaF** | sodium fluoride | 0.24 | 0.24 | 0.24 | 0.24 | 0.24 |
| **Silica HCS DP-105** | silica abrasive | 16 | 16 | 20 | 20 | 15 |
| **A200 silica** | fumed silica | 3 | - | 1.5 | 1.0 | - |
| **x-PVP** | cross-linked PVP polymer | - | 3 | - | - | - |
| **Gennencor (oxy Go HP L5000)** | glucose oxidase | 0.25 | 0.5 | 0.5 | 0.5 | 0.25 |
| **Staleydex 333 Dextrose** | glucose | 1.5 | 3.0 | 3 | 3 | 3 |
| **SLS** | SLS powder | - | 1.20 | 1.2 | 1.2 | 1.2 |
| **Flavor 89-274** | Brite Crystal flavor | 0.95 | 0.95 | 0.95 | 0.95 | 0.95 |
| **Q7-9120 Fluid** | silicone fluid | - | - | 5.00 | 5 | 5 |
| **TSPP** | tetrasodium pyrophosphate | - | - | 2 | 2 | 2 |
| **Plastigel** | gelled polyethylene | - | - | - | 5 | 8.25 |
| **Bio PSA 8-7016** | silicone adhesive | - | - | - | 1 | 0.5 |
| | **Total** | **100** | **100** | **100** | **100** | **100** |

## Claims

1. A stable, non-aqueous dentifrice composition comprising
(i) at least one enzyme;
(ii) at least one substrate for the at least one enzyme;
and a suitable carrier, wherein the at least one substrate reacts under aqueous conditions in the presence of the at least one enzyme to generate hydrogen peroxide, and wherein the composition further comprises at least one polymer or copolymer, wherein the polymer or copolymer is polyethylene glycol, a PEG/PPG copolymer, PVP, polyethylene.

2. The composition of claim 1 further comprising at least one abrasive.

3. The composition of claim 1 or claim 2 further comprising at least one fluoride source.

4. The composition of any preceding claim further comprising at least one surfactant.

5. The composition of any preceding claim, wherein the non-aqueous dentifrice composition is contained in a single container.

6. The composition of any preceding claim further comprising at least one vitamin, at least one further polymer, at least one flavoring agent; at least one further enzyme, at least one humectant, and/or at least one preservative and combinations thereof.

7. The composition of any preceding claim, wherein the enzyme is glucose oxidase.

8. The composition of any preceding claim, wherein the substrate is glucose.

9. The composition of claim 2, wherein the abrasive is one member chosen from abrasive silica, sodium metaphosphate, potassium metaphosphate, tricalcium phosphate, dihydrated dicalcium phosphate, aluminum silicate, calcined alumina, bentonite or other siliceous materials, or combinations thereof.

10. The composition of claim 3, wherein the fluoride source is one member chosen from sodium fluoride, potassium fluoride, sodium fluorosilicate, sodium monfluorophosphate (MFP), ammonium fluorosilicate, stannous fluoride and stannous chloride.

11. The composition of claim 6, wherein the flavoring agent is one member chosen from oil of wintergreen, oil of peppermint, oil of spearmint, oil of sassafras, oil of clove, aspartame, acesulfame, saccharin, dextrose, levulose and sodium cyclamate and combinations thereof.

12. A method of whitening a tooth surface comprising contacting the tooth surface with a stable, non-aqueous dentifrice composition according to any preceding claim.

13. A stable, non-aqueous dentifrice composition according to any of claims 1 to 11, for use in the treatment of oral calculus accumulation.

## Patentansprüche

1. Stabile, nichtwässrige Zahnreinigungsmittel-Zusammensetzung, umfassend
(i) mindestens ein Enzym;
(ii) mindestens ein Substrat für das mindestens eine Enzym;
und einen geeigneten Träger, wobei das mindestens eine Substrat unter wässrigen Bedingungen in der Gegenwart des mindestens einen Enzyms reagiert, um Wasserstoffperoxid zu erzeugen, und wobei die Zusammensetzung weiterhin mindestens ein Polymer oder Copolymer umfasst, wobei das Polymer oder Copolymer Polyethylenglycol, ein PEG/PPG-Copolymer, PVP, Polyethylen ist.

2. Die Zusammensetzung nach Anspruch 1, weiterhin umfassend mindestens einen abrasiven Stoff.

3. Die Zusammensetzung nach Anspruch 1 oder Anspruch 2, weiterhin umfassend mindestens eine Fluorid-Quelle.

4. Die Zusammensetzung nach einem beliebigen vorangegangenen Anspruch, weiterhin umfassend mindestens ein oberflächenaktives Mittel.

5. Die Zusammensetzung nach einem beliebigen vorangegangenen Anspruch, wobei die nichtwässrige Zahnreinigungsmittel-Zusammensetzung in einem Einzelbehälter enthalten ist.

6. Die Zusammensetzung nach einem beliebigen vorangegangenen Anspruch, weiterhin umfassend mindestens ein Vitamin, mindestens ein weiteres Polymer, mindestens einen Aromastoff; mindestens ein weiteres Enzym, mindestens ein Feuchthaltemittel und/oder mindestens ein Konservierungsmittel und Kombinationen davon.

7. Die Zusammensetzung nach einem beliebigen vorangegangenen Anspruch, wobei das Enzym Glucoseoxidase ist.

8. Die Zusammensetzung nach einem beliebigen vorangegangenen Anspruch, wobei das Substrat Glucose ist.

9. Die Zusammensetzung nach Anspruch 2, wobei der abrasive Stoff ein Mitglied ist, ausgewählt aus abrasivem Silica, Natriummetaphosphat, Kaliummethaphosphat, Tricalciumphosphat, dihydratisiertes Dicalciumphosphat, Aluminiumsilicat, kalziniertes Aluminiumoxid, Bentonit oder anderen silikatischen Materialien, oder Kombinationen davon.

10. Die Zusammensetzung nach Anspruch 3, wobei die Fluorid-Quelle ein Mitglied ist, ausgewählt aus Natriumfluorid, Kaliumfluorid, Natriumfluorsilicat, Natriummonofluorphosphat (MFP), Ammoniumfluorsilicat, Zinnfluorid und Zinnchlorid.

11. Die Zusammensetzung nach Anspruch 6, wobei das Aromamittel ein Mitglied ist, ausgewählt aus einem Öl von Wintergrün, einem Öl von Pfefferminze, einem Öl von grüner Minze, einem Öl von Sassafras, einem Nelkenöl, Aspartam, Acesulfam, Saccharin, Dextrose, Levulose und Natriumcyclamat und Kombinationen davon.

12. Verfahren zum Aufhellen einer Zahnoberfläche, umfassend das InKontaktbringen der Zahnoberfläche mit einer stabilen, nichtwässrigen Zahnreinigungsmittel-Zusammensetzung gemäß einem beliebigen vorangegangenen Anspruch.

13. Stabile, nichtwässrige Zahnreinigungsmittel-Zusammensetzung gemäß einem beliebigen der Ansprüche 1 bis 11, zur Verwendung bei der Behandlung von oraler Zahnstein-Ansammlung.

## Revendications

1. Composition de dentifrice non aqueuse, stable, comprenant
(i) au moins une enzyme ;
(ii) au moins un substrat pour l'enzyme ou les enzymes ;
et un support approprié, dans laquelle le ou les substrats réagissent dans des conditions aqueuses en présence de l'enzyme ou des enzymes pour générer du peroxyde d'hydrogène, et dans laquelle la composition comprend en outre au moins un polymère ou un copolymère, dans laquelle le polymère ou le copolymère est un polyéthylène glycol, un copolymère de PEG/PPG, le PVP, le polyéthylène.

2. Composition selon la revendication 1, comprenant en outre au moins un abrasif.

3. Composition selon la revendication 1 ou la revendication 2, comprenant en outre au moins une source de fluorure.

4. Composition selon l'une quelconque des revendications précédentes, comprenant en outre au moins un tensioactif.

5. Composition selon l'une quelconque des revendications précédentes, dans laquelle la composition de dentifrice non aqueuse est contenue dans un seul récipient.

6. Composition selon l'une quelconque des revendications précédentes, comprenant en outre au moins une vitamine, au moins un polymère supplémentaire, au moins un agent aromatisant ; au moins une enzyme supplémentaire, au moins un humectant, et/ou au moins un conservateur et des combinaisons de ceux-ci.

7. Composition selon l'une quelconque des revendications précédentes, dans laquelle l'enzyme est la glucose oxydase.

8. Composition selon l'une quelconque des revendications précédentes, dans laquelle le substrat est le glucose.

9. Composition selon la revendication 2, dans laquelle l'abrasif est un élément choisi parmi une silice abrasive, le métaphosphate de sodium, le métaphosphate de potassium, le phosphate tricalcique, le phosphate dicalcique dihydraté, le silicate d'aluminium, l'alumine calcinée, la bentonite ou d'autres matériaux siliceux, ou des combinaisons de ceux-ci.

10. Composition selon la revendication 3, dans laquelle la source de fluorure est un élément choisi parmi le fluorure de sodium, le fluorure de potassium, le fluorosilicate de sodium, le monofluorophosphate de sodium (MFP), le fluorosilicate d'ammonium, le fluorure stanneux et le chlorure stanneux.

11. Composition selon la revendication 6, dans laquelle l'agent aromatisant est un élément choisi parmi l'essence de wintergreen, l'essence de menthe poivrée, l'essence de menthe verte, l'essence de sassafras, l'essence de clou de girofle, l'aspartame, l'acésulfame, la saccharine, le dextrose, le lévulose et le cyclamate de sodium et les combinaisons de ceux-ci.

12. Procédé de blanchiment d'une surface dentaire, comprenant la mise en contact de la surface dentaire avec une composition de dentifrice non aqueuse, stable selon l'une quelconque des revendications précédentes.

13. Composition de dentifrice non aqueuse, stable selon l'une quelconque des revendications 1 à 11, pour une utilisation dans le traitement de l'accumulation buccale de tartre.
